# EUROPÄISCHE PATENTSCHRIFT

(11) **EP 0 865 274 B1**
(45) Veröffentlichungstag und Bekanntmachung des Hinweises auf die Patenterteilung: **29.09.1999**
(21) Anmeldenummer: 96942341.7
(22) Anmeldetag: 04.12.1996
(51) Int. Cl.: A61K 9/70

(54) **FLÄCHENSTABILISIERTE PHARMAZEUTISCHE ZUBEREITUNG ZUR ANWENDUNG AUF DER HAUT**
SURFACE-STABILISED PHARMACEUTICAL PREPARATION FOR APPLICATION ON THE SKIN
PREPARATION PHARMACEUTIQUE A SURFACE STABILISEE POUR APPLICATION SUR LA PEAU

(30) Priorität: 09.12.1995 DE 19546024
(43) Veröffentlichungstag der Anmeldung: 23.09.1998
(73) Patentinhaber: LTS LOHMANN Therapie-Systeme GmbH, 56567 Neuwied (DE)
(72) Erfinder: HORSTMANN, Michael, D-56564 Neuwied (DE); LAUX, Wolfgang, D-65582 Dietz (DE)
(74) Vertreter: Flaccus, Rolf-Dieter, Dr.
(86) Internationale Anmeldenummer: EP9605411
(87) Internationale Veröffentlichungsnummer: WO9721430

(56) Entgegenhaltungen:
- WO-A-89/11262
- WO-A-92/09252
- US-A- 4 725 439
- US-A- 5 246 705

## Beschreibung

Die Erfindung betrifft eine pharmazeutische Zubereitung zur Abgabe von Wirkstoffen durch die Haut in den menschlichen Körper.

Bereits seit dem Mittelalter ist es bekannt, daß pharmazeutische Wirkstoffe durch die menschliche Haut hindurchwandern und somit durch Auflegen oder Aufstreichen entsprechender Zubereitungen pharmazeutischer Wirkstoffe auf die Haut arzneiliche Wirkungen hervorgerufen werden können. Diese Wirkungen sind nicht auf die Haut und das darunterliegende Gewebe beschränkt, sondern können sich bei entsprechend geeigneten Substanzen auf fernliegende Organe erstrecken, da die Wirkstoffe nach ihrer Aufnahme über den Blutkreislauf verteilt werden. Auf dem Markt befinden sich zum Beispiel hormonhaltige Salben und Cremes. Weiterhin sind auch antirheumatisch wirkende halbfeste Zubereitungen im Handel, welche nach Aufstreichen auf die Haut über lokale oder systemische Effekte eine therapeutische Wirkung auslösen. Dabei bestimmt nicht nur die Wirkstoffkonzentration das Ausmaß der Wirkstoffaufnahme, sondern auch unter anderem die schwierig zu kontrollierende Größe der behandelten Hautfläche. Auch die den Therapieerfolg mitbestimmende Schichtdicke der aufgetragenen Zubereitung kann vom Anwender kaum genau eingestellt werden. Daher sind therapeutische Maßnahmen mit solchen Formen mit nicht zu tolerierenden Mängeln behaftet.
Transdermale therapeutische Systeme (TTS), welche unter anderem neben der Wirkstoffkonzentration auch die Applikationsfläche genau definieren, weisen diesen Nachteil nicht auf.Zwischen den z.Zt. bekannten TTS gibt es einige grundsätzliche Gemeinsamkeiten:
1. Zum Schutz vor unerwünschter Abgabe von flüchtigen Bestandteilen des TTS nach außen, des weiteren auch zum Schutz vor nachteiligen Auswirkungen bei Kontakt mit anderen Flächen und nicht zuletzt zur Gewährleistung der mechanischen Stabilität des TTS wird eine für die Bestandteile des TTS undurchlässige, nicht klebende Rückschicht verwendet.
2. Da TTS in innigem Kontakt auf der Haut haften sollen, wird die der Haut zugewandte Schichtzumindest teilweise selbstklebend ausgerüstet.
3. Wegen dieser selbstklebenden Eigenschaften ist zur Lagerung vor Gebrauch eine dehäsiv ausgerüstete ablösbare Schutzschicht aufgebracht.

Die Rückschicht besteht in der Regel aus geeigneten Materialien wie Kunststoffolien, aber auch Papiere, Vliesstoffe oder Textilien kommen in Frage.

Die obengenannten TTS können trotz des gewonnenen Fortschrittes einige Nachteile aufweisen. So erzeugt beispielsweise eine flächenstabilisierende wirkstoffundurchlässige Rückschicht eine Einschränkung des Tragekomforts beim Patienten. Durch Starrheit verwendeter Folien können sich unerwünschte Limitierungen der Flächen ergeben. Da für eine Anzahl von pharmazeutischen Wirkstoffen die flächenbezogene Transportrate durch die Haut relativ gering sein kann, ist jedoch eine größere Systemfläche wünschenswert, um auch für solche Wirkstoffe genügend wirkungsvolle TTS bereitzustellen.

Es gab zwar Versuche, durch Verwendung elastischer Materialien für die Rückschicht den Tragekomfort zu verbessern (z.B. US 5,246,705), man beschränkte sich hier jedoch ausdrücklich auf Materialien, die für den Arzneistoff undurchlässig waren.
Ein weiterer Nachteil der dem Stand der Technik entsprechenden TTS ist fallweise ihre für den Patienten subjektiv als störend empfundene Dicke. Da nach konventioneller Aufbau im wesentlichen nur die Matrix, Klebschicht oder eventuell vorhandene Reservoirschichten zur Speicherung und Abgabe des Wirkstoffes genutzt werden, hat eine definitionsgemäß wirkstoffundurchlässige Rückschicht in dieser Hinsicht keine Funktion.
Aufgabe der Erfindung ist die Bereitstellung einer hauthaftenden pharmazeutischen Zubereitung mit vorgegebener Größe der Auflagefläche, die eine nichtklebrige Rückschicht und ein aus einer hauthaftenden Klebschicht gebildetes wirkstoffhaltiges Reservoir umfasst und die Nachteile des Standes der Technik nicht aufweist.

Diese Aufgabe wird erfindungsgemäß dadurch gelöst, daß die Rückschicht mindestens ein Drittel der in der Zubereitung insgesamt enthaltenen Wirkstoffmenge enthält.
Mit Vorteil hat das Einbeziehen der Rückschicht in den Diffusionsprozeß zur Folge, daß die Rückschicht im erfindungsgemäßen System eine Doppelfunktion aufweist: Sie bildet einerseits einen Teil des Wirkstoffreservoirs und andererseits eine Rückschicht, die ein Ankleben des Pflasters an Textilien und andere Gegenstände verhindert. Überraschend erwies sich dabei, daß die unvermeidliche Wirkstoffdurchlässigkeit im Regelfall dennoch keinen Nachteil darstellt, da nur im Falle flüchtiger Wirk- und/oder Hilfsstoffe ein merklicher Verlust über die Rückschicht an die Umgebung stattfindet.

Als Beispiele für solche pharmazeutische Wirkstoffe sind Nicotin oder Nitroglycerin zu nennen. Ethanol, Propandiol und andere niedermolekulare Alkohole, Menthol, Eucalyptol, Limonen und viele andere Terpene, niedermolekulare Fettsäuren wie z.B. Caprinsäure, Dimethylsulfoxid seien exemplarisch für typische Zusatzstoffe in solchen Zubereitungen genannt, die mehr oder weniger stark durch die Rückschicht aus der Zubereitung herauswandern. Unerwarteterweise erweist sich jedoch ein häufig anzutreffender Vorbehalt (z.B. EP 0 366 240, S. 3, Z. 24, oder EP 0 402 407, S. 5, Z. 4) gegen eine wirkstoffdurchlässige Rückschicht im wesentlichen als unbegründet, da weit mehr Wirkstoff von der Rückschicht über die Klebschicht zur Haut hin wandert als nach außen.

Infolge der geringen Dicke sowie der Möglichkeit, sehr flexible Materialien als Grundmaterial der Rückschicht zu verwenden, sind die erfindungsgemäßen Zubereitungen neben der anderen Funktionalität auch hinsichtlich des Aussehens von anderen TTS klassischer Bauart deutlich unterschieden. Da feine Hautfalten von der Zubereitung nachvollzogen werden, entsteht der Eindruck einer dünnen Schicht auf der Haut im Unterschied zu einer starren Klebefolie. Insofern nimmt der Erfindungsgegenstand eine Zwischenform zwischen einem TTS und einer auf einem Hautareal aufgetragenen Salbe ein. Durch ein Herstellungsverfahren, bei welchem ein wesentlicher Teil des Wirkstoffes bereits bei Fertigung in die Rückschicht eingebracht wird, werden Wanderungsvorgänge, die unter Umständen durch Quellungsvorgänge zu Faltenbildung führen, vermieden.

Zahlreiche Materialien sind für eine solche Rückschicht geeignet: Polyvinylalkohol, Styrol-Dien-Block-Copolymere, Polyurethane, Polyvinylchlorid, Polymethacrylate und viele andere Stoffe sind grundsätzlich geeignet, um nur einige Beispiele zu nennen.

Bei einer vorteilhaften Ausführungsform ist die Rückschicht durch eine vollflächig auf dieser haftende, dehäsiv ausgerüstete, nach Applikation abnehmbare Stützschicht abgedeckt.
Als pharmazeutische Wirkstoffe sind vorteilhaft Steroidhormone, Wirkstoffe mit Wirkung auf das Zentralnervensystem oder die Alzheimersche Krankheit, Antirheumatika oder Acetylsalicylsäure einsetzbar.

### Beispiele:

### Beispiel 1:

A) 10 g Styrol-Isopren-Styrol-Copolymer (Cariflex ® TR 1107) werden in 20 g Benzin mit einem Siedebereich zwischen 80 und 100 °C vollständig gelöst.
   100 mg 17-β-Estradiol, gelöst in 5 g Ethylacetat, werden hinzugefügt und die Masse wird nach homogener Vermischung in einer Spaltbreite von ca. 150 µm so auf eine dehäsiv ausgerüstete Polyesterfolie beschichtet, daß nach 4-stündiger Trocknung bei 35 °C eine gleichmäßige Schicht mit einem Flächengewicht von 30 g/m² entsteht.
B) In einem getrennten Vorgang wird eine Lösung von 20 g hydriertem Kolophoniumglycerolesterharz (Staybelite Ester ® 5E) und 8 g Styrol-Isopren-Styrol-Copolymer (Cariflex ® TR 1107) in 10 g Ethylacetat und 10 g Benzin mit einem Siedebereich zwischen 80 und 100 °C angefertigt, in die 0,12 g 17-β-Estradiol eingetragen werden, die sich bei Raumtemperatur vollständig auflösen. Die Masse wird in einer Spaltbreite von ca. 100 µm so auf eine dehäsiv ausgerüstete Polyesterfolie beschichtet, daß nach halbstündiger Trocknung bei 35 °C und 15-minütiger Nachtrocknung bei 60°C eine gleichmäßige Schicht mit einem Flächengewicht von 23 g/m² entsteht.

Die so hergestellten Schichten aus Phase A (Rückschicht) und Phase B (Reservoir) werden durch Kaschieren aufeinandergebracht und haften spontan zu einem manuell nicht lösbaren Verbund. Die dehäsiv ausgerüstete Polyesterfolie aus Phase B und die Klebschicht sowie die Rückschicht werden in dem Fachmann bekannter Weise in einer der geometrischen Form der Zubereitung entsprechenden Kontur gestanzt und die außenliegenden Reste entfernt.
Die Arzneiform wird einzeln in Siegelbeutel verpackt.

Der Anwender entnimmt das Arzneimittel aus der Verpackung, entfernt die Schutzschicht (Release Liner) von der Klebschicht, klebt die Arzneiform auf eine geeignete Hautstelle und entfernt zuletzt von der Rückschicht die als Stützschicht verwendete dehäsiv ausgerüstete Polyesterfolie.

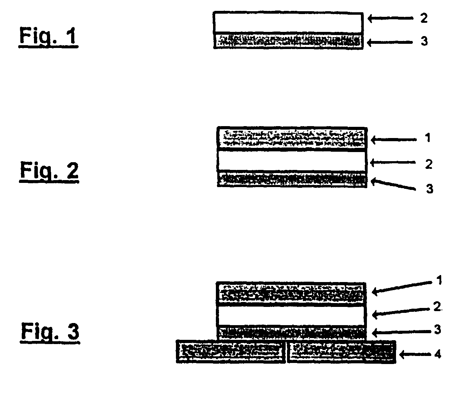

Nachfolgend wird der Aufbau einer erfindungsgemäßen Zubereitung anhand von Ausführungsbeispielen gemäß Figuren 1 bis 3 näher erläutert.
Fig. 1 zeigt den grundsätzlichen Aufbau des transdermalen Pflasters im Schnitt mit der wirkstoffhaltigen und wirkstoffdurchlässigen Rückschicht (2) und dem mit einer Klebschicht (3) ausgebildeten wirkstoffhaltigen Reservoir.
Fig. 2 zeigt das Pflaster gemäß Fig. 1, jedoch mit der auf der Rückschicht (2) vollständig haftenden Stützschicht (1).
Fig. 3 zeigt das Pflaster gemäß Fig. 1 und Fig. 2, jedoch zusätzlich mit vollständiger Abdeckung der Klebschicht (3) mit einer kleberabweisenden, abnehmbaren Stützschicht (4), (Release liner) für die Klebschicht (3).

## Patentansprüche

1. Wirkstoffhaltige transdermale pharmazeutische Zubereitung mit vorgegebener Größe der Auflagefläche, umfassend eine nichtklebrige wirkstoffdurchlässige Rückschicht und eine hauthaftende Klebschicht, dadurch gekennzeichnet, daß die Rückschicht mindestens ein Drittel der in der Zubereitung insgesamt enthaltenen Wirkstoffmenge enthält.

2. Pharmazeutische Zubereitung nach Anspruch 1, dadurch gekennzeichnet, daß die Rückschicht durch eine vollflächig auf dieser haftende, dehäsiv ausgerüstete, nach Applikation abnehmbare Stützschicht abgedeckt ist.

3. Pharmazeutische Zubereitung nach Anspruch 1 oder 2, dadurch gekennzeichnet, daß die Klebschicht durch eine vollflächig auf dieser haftende, vor Applikation auf die Haut abnehmbare, kleberabweisende Schutzschicht abgedeckt ist.

4. Pharmazeutische Zubereitung nach einem oder mehreren der vorhergehenden Ansprüche, dadurch gekennzeichnet, daß die Klebschicht und die Rückschicht zusammen eine Dicke von weniger als 80 µm, bevorzugt weniger als 40 µm aufweist.

5. Pharmazeutische Zubereitung nach einem oder mehreren der vorhergehenden Ansprüche, dadurch gekennzeichnet, daß sie als pharmazeutischen Wirkstoff ein Steroidhormon enthält.

6. Pharmazeutische Zubereitung nach einem oder mehreren der Ansprüche 1 bis 4, dadurch gekennzeichnet, daß sie als pharmazeutischen Wirkstoff einen solchen mit Wirkung auf das Zentralnervensystem enthält.

7. Pharmazeutische Zubereitung nach einem oder mehreren der Ansprüche 1 bis 4, dadurch gekennzeichnet, daß sie als pharmazeutischen Wirkstoff einen solchen mit Wirkung auf die Alzheimersche Erkrankung enthält.

8. Pharmazeutische Zubereitung nach einem oder mehreren der Ansprüche 1 bis 4, dadurch gekennzeichnet, daß sie als pharmazeutischen Wirkstoff ein Antirheumatikum enthält.

9. Pharmazeutische Zubereitung nach einem oder mehreren der Ansprüche 1 bis 4, dadurch gekennzeichnet, daß sie als pharmazeutischen Wirkstoff Acetylsalicylsäure enthält.

10. Verfahren zur Herstellung der transdermalen pharmazeutischen Zubereitung nach den vorhergehenden Ansprüchen, dadurch gekennzeichnet, daß in einem ersten Arbeitsschritt ein als Grundmaterial verwendetes Polymer durch Zusatz von Lösemitteln in Lösung gebracht oder durch Erhitzen daraus eine Schmelze bereitet und darin mindestens ein Drittel der vorgesehenen Wirkstoffmenge eingetragen, homogen vermischt, mit der Mischung die dehäsiv ausgerüstete Stützfolie in gleichmäßiger Schicht beschichtet und diese getrocknet wird, und daß in einem separaten Arbeitsschritt eine den Rest des Wirkstoffe enthaltende, streichfähige Klebemasse ebenfalls auf die dehäsiv ausgerüstete Schutzfolie beschichtet und getrocknet wird und die so hergestellten Schichten aufeinanderlaminiert und durch Formstanzen Pflaster gebildet werden.

## Claims

1. An active substance-containing transdermal pharmaceutical preparation with a given size of contact surface, comprising a tack-free backing layer which is permeable to active substances and an adhesive layer adhering to the skin, characterized in that the backing layer comprises at least a third of the total active substance amount contained in the preparation.

2. The pharmaceutical preparation according to claim 1 characterized in that the backing layer is covered by a supporting layer which adheres to the whole surface thereof, has been rendered antiadhesive, and is removable after application.

3. The pharmaceutical preparation according to claim 1 or 2 characterized in that the adhesive layer is covered by a nonstick protective layer which adheres to the whole surface thereof and is removable prior to application to the skin.

4. The pharmaceutical preparation according to one or several of the preceding claims characterized in that the adhesive layer and the backing layer together have a thickness of less than 80 µm, preferably less than 40 µm.

5. The pharmaceutical preparation according to one or several of the preceding claims characterized in that it comprises a steroid hormone as pharmaceutical active substance.

6. The pharmaceutical preparation according to one or several of claims 1 to 4 characterized in that it comprises as pharmaceutical active substance one having an effect on the central nervous system.

7. The pharmaceutical preparation according to one or several of claims 1 to 4 characterized in that it comprises as pharmaceutical active substance one having an effect on the Alzheimer's disease.

8. The pharmaceutical preparation according to one or several of claims 1 to 4 characterized in that it comprises an antirheumatic as pharmaceutical active substance.

9. The pharmaceutical preparation according to one or several of claims 1 to 4 characterized in that it comprises acetylsalicylic acid as pharmaceutical active substance.

10. A process for the production of the transdermal pharmaceutical preparation according to the preceding claims, characterized in that, in a first step, a polymer used as base material is brought into solution by adding solvents or is made into a melt by heating, at least a third of the intended active substance amount is added and homogeneously mixed, the antiadhesive supporting film is coated with the mixture to form a uniform layer which is dried, and that, in a separate step, a spreadable adhesive mass comprising the remaining active substance amount is also coated on the antiadhesive protective film and dried, that the layers so produced are laminated on top of each other, and that patches are formed by punching.

## Revendications

1. Formulation pharmaceutique transdermique contenant un principe actif, dont la surface d'appui possède une dimension prédéfinie, comprenant une couche dorsale non collante perméable au principe actif et une couche adhésive adhérant à la peau, caractérisée en ce que la couche dorsale contient au moins un tiers de la quantité de principe actif contenu au total dans la formulation.

2. Formulation pharmaceutique selon la revendication 1, caractérisée en ce que la couche dorsale est recouverte par une couche de support adhérant sur toute la surface de la première citée, dont le côté externe a été rendu antiadhésif et qui peut être pelliculée après l'application.

3. Formulation pharmaceutique selon la revendication 1 ou 2, caractérisée en ce que la couche adhésive est recouverte par une couche de protection antiadhésive, adhérant sur toute la surface de la couche adhésive et qui peut être pelliculée avant l'application sur la peau.

4. Formulation pharmaceutique selon une ou plusieurs des revendications précédentes, caractérisée en ce que là couche adhésive et la couche dorsale présentent ensemble une épaisseur inférieure à 80 µm, de préférence inférieure à 40 µm.

5. Formulation pharmaceutique selon une ou plusieurs des revendications précédentes, caractérisée en ce qu'elle contient, à titre de principe actif pharmaceutique, une hormone stéroïdienne.

6. Formulation pharmaceutique selon une ou plusieurs des revendications 1 à 4, caractérisée en ce qu'elle contient, à titre de principe actif pharmaceutique, un principe actif ayant un effet sur le système nerveux central.

7. Formulation pharmaceutique selon une ou plusieurs des revendications 1 à 4, caractérisée en ce qu'elle contient, à titre de principe actif pharmaceutique, un principe actif ayant un effet sur la maladie d'Alzheimer.

8. Formulation pharmaceutique selon une ou plusieurs des revendications 1 à 4, caractérisée en ce qu'elle contient, à titre de principe actif pharmaceutique, un agent anti-rhumatismal.

9. Formulation pharmaceutique selon une ou plusieurs des revendications 1 à 4, caractérisée en ce qu'elle contient, à titre de principe actif pharmaceutique, de l'acide acétylsalicylique.

10. Procédé pour la préparation de la formulation pharmaceutique transdermique selon les revendications précédentes, caractérisé en ce qu'on amène en solution, dans une première étape opératoire, un polymère utilisé comme matière de base, par addition de solvants, ou bien on prépare une masse fondue par réchauffement de cette dernière et on y introduit au moins 1/3 de la quantité prévue de principe actif, on mélange intimement pour obtenir un mélange homogène, on enduit avec le mélange la feuille de support dont le côté externe a été rendu anti-adhésif, pour obtenir une couche uniforme, puis on sèche cette dernière, et en ce que, dans une étape opératoire séparée, on enduit une matière adhésive contenant le reste du principe actif et apte à l'enduction, également sur la feuille de protection dont le côté externe a été rendu anti-adhésif et on la sèche, puis on contrecolle les unes aux autres les couches ainsi préparées et on forme des emplâtres par nervurage à la presse.
